# EUROPEAN PATENT SPECIFICATION

(11) **EP 2 335 697 B1**
(45) Date of publication and mention of the grant of the patent: **12.03.2014**
(21) Application number: 09813076.8
(22) Date of filing: 09.09.2009
(51) Int. Cl.: A61K 9/20, A61K 9/16, A61K 31/195, A61K 31/197, A61K 31/375, A61K 31/198, A61K 31/4415

(54) **STABLE PHARMACEUTICAL FORMULATION WITH LIMITED DISCOLORATION**
STABILE PHARMAZEUTISCHE FORMULIERUNG MIT BEGRENZTER VERFÄRBUNG
PRÉPARATION MÉDICINALE STABLE DONT LA MODIFICATION DE COULEUR EST SUPPRIMÉE.

(30) Priority: 12.09.2008 JP 2008235354
(43) Date of publication of application: 22.06.2011
(73) Proprietor: Daiichi Sankyo Healthcare Co., Ltd., Chuo-ku Tokyo 103-8541 (JP)
(72) Inventor: MATSUSHITA, Takeshi, Chuo-ku Tokyo 103-8541 (JP); MOCHIZUKI, Yusuke, Chuo-ku Tokyo 103-8541 (JP)
(74) Representative: Oldroyd, Richard Duncan
(86) International application number: PCT/JP2009/065700
(87) International publication number: WO 2010/029930

(56) References cited:
- EP-A1- 1 657 265
- WO-A1-2005/019286
- WO-A1-2006/003965
- JP-A- 9 003 348
- JP-A- 2004 250 376
- JP-A- 2005 314 403
- JP-A- 2007 197 410
- JP-A- 2008 201 711

## Description

### [Technical Field]

The present invention relates to a solid pharmaceutical preparation for oral administration which is less likely to be discolored even if the preparation contains tranexamic acid and ascorbic acid.

### [Background Art]

Tranexamic acid has an anti-bleeding effect (as an antiplasmin), an antiallergic effect, an antiinflammatory effect and the like, is widely used as ethical drugs and is incorporated also in OTC drugs. After it was reported in 1979 that when tranexamic acid was administered to a patient with chronic urticaria, chloasma which happened to occur at the same time in the same patient disappeared, tranexamic acid began to be prescribed for the treatment of chloasma (see, for example, Non Patent Literature 1).

It has been known for a long time that ascorbic acid suppresses the production of melanin pigment, and that due to the action of degrading melanin pigment which has been already accumulated, ascorbic acid also suppresses pigmentation. As for the OTC drugs, in the approved indications of preparations containing vitamin C as a base component, flecks, freckles, and pigmentation caused by sunburn or rash are included (see Non Patent Literature 2).

Further, a preparation for treatment of pigmentation containing tranexamic acid and ascorbic acid has been disclosed, and is reported to have superior degree of improvement of pigmentation than the case where tranexamic acid or ascorbic acid is administered as a single drug (see Patent Literature 1).

Further, a skin whitening composition containing tranexamic acid, ascorbic acid and L-cysteine has been disclosed by the present inventors, and is found to suppress pigmentation further than the concomitant use of tranexamic acid and ascorbic acid (see Patent Literature 2). In the preparation example of Patent Literature 2, a tablet containing, in addition to the above-mentioned three components serving as active ingredients, crystalline cellulose, cornstarch, hydroxypropyl cellulose with low-substitution levels, hydroxypropyl cellulose, magnesium stearate, hypromellose, macrogol, talc and titanium oxide as additives is disclosed.

Further, in the preparation example of Patent Literature 3, a tablet containing, in addition to tranexamic acid, ascorbic acid, L-cysteine, pyridoxine hydrochloride and calcium pantothenate as active ingredients, crystalline cellulose, cornstarch, hydroxypropyl cellulose with low-substitution levels, hydroxypropyl cellulose, magnesium stearate, hypromellose, macrogol, talc and titanium oxide as pharmaceutical additives is disclosed (see Patent Literature 3) .

On the other hand, a polyvinyl alcohol copolymer (PVA copolymer) which is obtained by copolymerization of a polyvinyl alcohol with a polymerizable vinyl monomer has been used as a gelatin-free capsule material with the rise of the bovine spongiform encephalopathy (BSE) issue (see, for example, Patent Literature 4). In addition to a capsule, the medical use of PVA copolymer as a coating agent for a tablet or a granule (Patent Literature 5) or as a pharmaceutical binder (Patent Literature 6) is disclosed.

Further, a partially saponified polyvinyl alcohol which is obtained by saponification of polyvinyl acetate has a saponification degree of 78 to 96 mol%. As for the medical application, the partially saponified polyvinyl alcohol is used as a coating agent for a tablet or a granule, a pharmaceutical binder, or the like (see, for example, Non Patent Literature 3).

However, a preparation containing tranexamic acid, ascorbic acid and the like as active ingredients and further containing a polyvinyl alcohol as an additive has not yet been know, let alone for one further containing a polyvinyl alcohol copolymer and/or a partially saponified polyvinyl alcohol as extra additives.

### [Prior Art Literatures]

### [Patent Literatures]

[Patent Literature 1] JP-A-4-243825
[Patent Literature 2] JP-A-2004-217655
[Patent Literature 3] JP-A-2005-314403
[Patent Literature 4] JP-A-2007-091670
[Patent Literature 5] JP-A-2007-022938
[Patent Literature 6] WO 2005/019286

### [Non Patent Literatures]

[Non Patent Literature 1] Pharmacia Vol. 44, No. 5, 2008, pp. 437-442
[Non Patent Literature 2] Ippan-yo Iyakuhin Seizo (Yunyu) Shonin Kijun (Approval Standards for Manufacturing of Non-Prescription Drugs (Import)), Jiho, 2008
[Non Patent Literature 3] Iyakuhin Tenkabutsu Jiten (Japanese Pharmaceutical Excipients Directory) 2005, Yakuji Nippo Limited, 2005

### [Summary of Invention]

### [Problems that the Invention is to Solve]

The present inventors actually produced, on a trial basis, a granule containing tranexamic acid and ascorbic acid disclosed in Example 1 of Patent Literature 1 and recognized a problem that the granule gradually change its color (red discoloration) when it was subjected to a long-term strage (in an accelerated test at a temperature of 40°C and a relative humidity of 75%).

That is, an object of the invention is to provide a solid preparation for oral administration which contains tranexamic acid and ascorbic acid and is excellent in stability without causing discoloration.

### [Means for Solving the Problems]

In order to solve the above problems, the present inventors performed an extensive study with trials and errors to find which additive contributes the most to the stability of a preparation containing tranexamic acid and ascorbic acid as active ingredients.

As a result, the present inventors discovered a startling fact that when a polyvinyl alcohol copolymer and/or a partially saponified polyvinyl alcohol are/is added to the preparation containing tranexamic acid and ascorbic acid, discoloration of the preparation can be significantly prevented.

Further, the present inventors discovered that when a polyvinyl alcohol copolymer and/or a partially saponified polyvinyl alcohol are/is used as a granulation binder in the preparation of the invention, discoloration of the preparation can be significantly prevented, and thus completed the present invention.

That is, the invention is directed to:
(1) a solid pharmaceutical preparation for oral administration comprising tranexamic acid and ascorbic acid, wherein said solid pharmaceutical preparation further contains a polyvinyl alcohol copolymer and/or a partially saponified polyvinyl alcohol, and preferred embodiments of the invention are as shown below:
(2) the solid pharmaceutical preparation for oral administration described in (1), further containing L-cysteine;
(3) the solid pharmaceutical preparation for oral administration described in (1) or (2), further containing pantothenic acid and/or pyridoxine;
(4) the solid pharmaceutical preparation for oral administration described in (3), wherein the pyridoxine is pyridoxine hydrochloride; and
(5) the solid pharmaceutical preparation for oral administration described in (3) or (4), wherein the pantothenic acid is calcium pantothenate.

### [Advantage of the Invention]

The preparation of the present invention is stable and useful because its discoloration is prevented even if tranexamic acid and ascorbic acid are incorporated therein. Further, the invention is useful because it can be used to obtain a stable preparation wherein the discoloration is prevented even if active ingredients such as L-cysteine, pyridoxine hydrochloride and calcium pantothenate are further incorporated in the preparation.

### [Best Mode for Carrying Out the Invention]

The "pyridoxine" in the invention includes pyridoxine and a salt thereof , and is preferably pyridoxine hydrochloride.

The "pantothenic acid" in the invention includes pantothenic acid and a salt thereof, and is preferably calcium pantothenate.

Tranexamic acid, ascorbic acid, calcium pantothenate and pyridoxine hydrochloride in the invention are listed in the Japanese Pharmacopoeia Fifteenth Edition, respectively.

L-cysteine and the partially saponified polyvinyl alcohol in the invention are listed in, for example, Japan Pharmaceutical Excipients 2003.

The "partially saponified polyvinyl alcohol" in the invention is a partially saponified polyvinyl acetate polymer obtained by polymerization of vinyl acetate and generally has a saponification degree of 78 to 96 mol% and preferably has a viscosity of 2 to 100 mm²/s.

The partially saponified polyvinyl alcohol to be used in the invention is commercially available under a product name "Gosenol" (The Nippon Synthetic Chemical Industry Co., Ltd.) and thus can be easily obtained.

Further, a film coating base containing a partially saponified polyvinyl alcohol is commercially available under a product name "Opadry II" (Colorcon Japan Limited) and thus can be easily obtained.

The "polyvinyl alcohol copolymer" for the invention can be produced according to the method described in WO 2005/19286 or WO 2002/17848, and is a polyvinyl alcohol copolymer which is obtained by copolymerization of a partially saponified polyvinyl alcohol having an average degree of polymerization of 100 to 2000 with at least one polymerizable vinyl monomer at a weight ratio of 6:4 to 9:1. Said copolymer has a viscosity of 10 to 300 mPa·s in the form of a 2% by weight solution at 20°C.

Preferred is a polyvinyl alcohol copolymer which is obtained by copolymerization of a partially saponified polyvinyl alcohol having an average degree of polymerization of 150 to 1000 with at least one polymerizable vinyl monomer at a weight ratio of 6:4 to 9:1, and a viscosity of 10 to 250 mPa·s in the form of a 2% by weight solution at 20°C.

As the polymerizable vinyl monomer to be used, acrylic acid, methacrylic acid, macrogol or the like is preferred.

Further, a copolymer of a polyvinyl alcohol and polyethylene glycol (macrogol) is also preferred as the polyvinyl alcohol copolymer to be used for the invention.

The polyvinyl alcohol copolymer to be used in the invention is commercially available, for example, under a product name "POVACOAT" (Nisshin Kasei Co. , Ltd.) or a product name "Kollicoat IR" (BASF) and can be easily obtained.

In the preparation of the invention, with respect to the content ratios of tranexamic acid, ascorbic acid and the polyvinyl alcohol copolymer or the partially saponified polyvinyl alcohol, the content ratios of ascorbic acid and the polyvinyl alcohol copolymer or the partially saponified polyvinyl alcohol based on 1 part by weight of tranexamic acid are from 0.01 to 10 parts by weight and 0.001 to 1 part by weight, respectively, preferably from 0.1 to 2 parts by weight and 0.01 to 0.5 part by weight, respectively.

The preparation of the invention contains tranexamic acid and ascorbic acid, and these components may exist in admixture or be separated by a boundary so as not to come into contact with each other. However, tranexamic acid and ascorbic acid preferably exist in the preparation as separate granules.

Next, a method for producing the solid pharmaceutical preparation of the invention will be described below.

A solid preparation in the invention refers to a granule, a pill, a powder, a tablet (including a multilayer tablet), a capsule and the like listed in the Japanese Pharmacopoeia Fifteenth Edition.

In the invention, the dosage form is preferably a granule or a tablet which may be coated with sugar or a film.

The solid pharmaceutical preparation of the invention can be produced using a known technique.

In the case where the dosage form of the solid pharmaceutical preparation of the invention is a granule or a tablet, the preparation can be produced using a known technique. Further, such a granule or a tablet can be coated with sugar or a film also by a known method.

### [Examples]

Hereinafter, Examples and Comparative Examples will be described for illustrating the invention in further detail. 1.1 Comparative Test in the Form of Casting Film

### (Comparative Example 1: Control)

An 8% aqueous solution of only hypromellose (also referred to as "hydroxypropylmethyl cellulose") was used as a control.

### (Comparative Example 2)

0.5 g of tranexamic acid and 0.2 g of ascorbic acid were dispersed or dissolved in 57.5 g of an 8% aqueous solution of hypromellose.

### (Example 1)

0.5 g of tranexamic acid and 0.2 g of ascorbic acid were dispersed or dissolved in 57.5 g of an 8% aqueous solution of a polyvinyl alcohol copolymer (product name "POVACOAT" manufactured by Nisshin Kasei Co., Ltd., omitted hereunder).

### (Example 2)

0.5 g of tranexamic acid and 0.2 g of ascorbic acid were dispersed or dissolved in 57.5 g of a 50% aqueous ethanol solution of a polyvinyl alcohol copolymer.

### (Example 3)

0.5 g of tranexamic acid and 0.2 g of ascorbic acid were dispersed or dissolved in 57.5 g of an 8% aqueous solution of a film coating base (product name "Opadry II") containing a partially saponified polyvinyl alcohol.

### (Example 4)

0.5 g of tranexamic acid and 0.2 g of ascorbic acid were dispersed or dissolved in 57.5 g of an 8% aqueous solution of a partially saponified polyvinyl alcohol.

### 1.2 Test Method

An appropriate amount of each of the samples of Examples 1 to 4 and Comparative Examples 1 to 2 was extracted and applied onto a white plate and left at 50°C for a whole day and night to prepare a casting film. Discoloration of each of the prepared casting films was visually observed. The evaluation of discoloration was performed in accordance with the following criteria:

### 1.3 Evaluation Criteria

A: no discoloration
B: slight discoloration
C: some discoloration
D: significant discoloration

### 1.4 Test Results

The test results are shown in Table 1. From the results shown in the table, it was found that discoloration can be prevented when granulation is performed using a polyvinyl alcohol copolymer and a partially saponified polyvinyl alcohol.

**(Table 1)**

| | Comparative Example 1 | Comparative Example 2 | Example 1 | Example 2 | Example 3 | Example 4 |
|---|---|---|---|---|---|---|
| Tranexamic acid | | ○ | ○ | ○ | ○ | ○ |
| Ascorbic acid | | ○ | ○ | ○ | ○ | ○ |
| Hypromellose | ○ | ○ | | | | |
| PVA copolymer | | | ○ | ○ | | |
| Partially saponified PVA | | | | | ○ | ○ |
| Visual evaluation | A | D | A | B | B | A |

### 2.1 Comparative Test as Granulation Binder

### (Comparative Example 3)

In a fluidized bed granulator (Flow Coater FLO-5, Freund Corporation), 2403.8 g of tranexamic acid, 118.6 g of calcium pantothenate and an appropriate amount of crystalline cellulose were placed and mixed, and then, an aqueous hypromellose solution was sprayed, whereby a granulated powder was prepared as "granule a".

Further, in the fluidized bed granulator, 961.5 g of ascorbic acid, 769.2 g of L-cysteine, 19.2 g of pyridoxine hydrochloride and an appropriate amount of crystalline cellulose were placed and mixed, and then, an aqueous hypromellose solution was sprayed, whereby a granulated powder was prepared as "granule b".

The thus prepared granules a and b were mixed, whereby a granulated granule was prepared. 3651.3 g of the granulated granule was mixed with 205.1 g of crystalline cellulose, 102.6 g of croscarmellose sodium and 41.0 g of magnesium stearate, and the resulting mixture was tableted, whereby a plain tablet serving as a central core was obtained.

### (Example 5)

In the fluidized bed granulator, 2403.8 g of tranexamic acid, 769.2 g of L-cysteine, 118.6 g of calcium pantothenate, 19.2 g of pyridoxine hydrochloride and an appropriate amount of crystalline cellulose were placed and mixed, and then, an aqueous polyvinyl alcohol copolymer (POVACOAT) solution was sprayed, whereby a granulated powder was prepared as "granule a".

Further, in the fluidized bed granulator, 961.5 g of ascorbic acid and an appropriate amount of crystalline cellulose were placed and mixed, and then, an aqueous polyvinyl alcohol copolymer (POVACOAT) solution was sprayed as a binder, whereby a granulated powder was prepared as "granule b".

The thus prepared granules a and b were mixed, whereby a granulated granule was prepared. 3651.3 g of the granulated granule was mixed with 205.1 g of crystalline cellulose, 102.6 g of croscarmellose sodium and 41.0 g of magnesium stearate, and the resulting mixture was tableted, whereby a plain tablet serving as a central core was obtained.

### (Example 6)

In the fluidized bed granulator, 2403.8 g of tranexamic acid, 118.6 g of calcium pantothenate and an appropriate amount of crystalline cellulose were placed and mixed, and then, an aqueous polyvinyl alcohol copolymer (POVACOAT) solution was sprayed, whereby a granulated powder was prepared as "granule a".

Further, in the fluidized bed granulator, 961.5 g of ascorbic acid, 769.2 g of L-cysteine, 19.2 g of pyridoxine hydrochloride and an appropriate amount of crystalline cellulose were placed and mixed, and then, an aqueous polyvinyl alcohol copolymer (POVACOAT) solution was sprayed as a binder, whereby a granulated powder was prepared as "granule b".

The thus prepared granules a and b were mixed, whereby a granulated granule was prepared. 3651.3 g of the granulated granule was mixed with 205.1 g of crystalline cellulose, 102.6 g of croscarmellose sodium and 41.0 g of magnesium stearate, and the resulting mixture was tableted, whereby a plain tablet serving as a central core was obtained.

### (Example 7)

In the fluidized bed granulator, 2403.8 g of tranexamic acid, 118.6 g of calcium pantothenate and an appropriate amount of crystalline cellulose were placed and mixed, and then, an aqueous partially saponified polyvinyl alcohol solution was sprayed, whereby a granulated powder was prepared as "granule a".

Further, in the fluidized bed granulator, 961.5 g of ascorbic acid, 769.2 g of L-cysteine, 19.2 g of pyridoxine hydrochloride and an appropriate amount of crystalline cellulose were placed and mixed, and then, an aqueous partially saponified polyvinyl alcohol solution was sprayed, whereby a granulated powder was prepared as "granule b".

The thus prepared granules a and b were mixed, whereby a granulated granule was prepared. 3651.3 g of the granulated granule was mixed with 205.1 g of crystalline cellulose, 102.6 g of croscarmellose sodium and 41.0 g of magnesium stearate, and the resulting mixture was tableted, whereby a plain tablet serving as a central core was obtained.

### (Example 8)

In the fluidized bed granulator, 386.6 g of tranexamic acid, 19. 1 g of calcium pantothenate, 20.6 g of nicotinamide, 30.9 g of 50-fold powder of tocopherol acetic acid ester (tocopherol acetate) and an appropriate amount of crystalline cellulose were placed and mixed, and then, an aqueous polyvinyl alcohol copolymer (POVACOAT) solution was sprayed, whereby a granulated powder was prepared as "granule a".

Further, in the fluidized bed granulator, 255.5 g of ascorbic acid, 204.4 g of L-cysteine, 10.2 g of pyridoxine hydrochloride and an appropriate amount of crystalline cellulose were placed and mixed, and then, an aqueous polyvinyl alcohol copolymer (POVACOAT) solution was sprayed, whereby a granulated powder was prepared as "granule b".

The thus prepared granules a and b were mixed, whereby a granulated granule was prepared. 741.4 g of the granulated granule was mixed with 30.5 g of crystalline cellulose, 20.0 g of croscarmellose sodium and 8.1 g of magnesium stearate, and the resulting mixture was tableted, whereby a plain tablet serving as a central core was obtained.

### 2.2 Test Method

60 tablets of each of the samples of Examples 5 to 8 and Comparative Example 3 were packed in a glass bottle (6K standard bottle) and stored at 25°C and 75% RH for 4 weeks. Then, discoloration thereof was visually observed. The evaluation of discoloration was performed according to the the following criteria.

### 2.3 Evaluation Criteria

In the same manner as in 1.3, the evaluation was performed according to the four criteria: A: no discoloration; B: slight discoloration; C: some discoloration; and D: significant discoloration.

### 2.4 Test Results

The test results are shown in Tables 2 and 3. From the results shown in the tables, it was found that discoloration can be prevented when granulation is performed using a polyvinyl alcohol copolymer and a partially saponified polyvinyl alcohol.

**(Table 2)**

| Component | Comparative Example 3 | | Example 5 | | Example 6 | |
|---|---|---|---|---|---|---|
| | Granule a | Granule b | Granule a | Granule b | Granule a | Granule b |
| Tranexamic acid | ○ | | ○ | | ○ | |
| Ascorbic acid | | ○ | | ○ | | ○ |
| L-cysteine | | ○ | ○ | | | ○ |
| Ca pantothenate | ○ | | ○ | | ○ | |
| Pyridoxine hydrochloride | | ○ | ○ | | | ○ |
| Hypromellose | ○ | ○ | | | | |
| PVA copolymer | | | ○ | ○ | ○ | ○ |
| Visual evaluation | C | | B | | B | |

**(Table 3)**

| Component | Example 7 | | Example 8 | |
|---|---|---|---|---|
| | Granule a | Granule b | Granule a | Granule b |
| Tranexamic acid | ○ | | ○ | |
| Ascorbic acid | | ○ | | ○ |
| L-cysteine | | ○ | | ○ |
| Ca pantothenate | ○ | | ○ | |
| Pyridoxine hydrochloride | | ○ | | ○ |
| Nicotinamide | | | ○ | |
| Tocopherol acetate | | | ○ | |
| PVA copolymer | | | ○ | ○ |
| Partially saponified PVA | ○ | ○ | | |
| Visual evaluation | B | | B | |

### 3.1 Comparative Test Using Known Preparation

The tablet disclosed in Preparation Example 3.2 of Patent Literature 3 was used. That is, as raw materials per 6 tablets, 750 mg of tranexamic acid, 300 mg of ascorbic acid, 240 mg of L-cysteine, 24 mg of calcium pantothenate, 6 mg of pyridoxine hydrochloride, an appropriate amount of crystalline cellulose and an appropriate amount of low-substituted hydroxypropylmethyl cellulose were mixed, and the resulting mixture was granulated using an aqueous hypromellose solution. Then, an appropriate amount of magnesium stearate was mixed therein, and the mixture was tableted, whereby a plain tablet serving as a central core was obtained.

### (Comparative Example 4)

The plain tablet was pulverized, and an appropriate amount of the pulverized tablet was dispersed or dissolved in an aqueous hypromellose solution.

### (Comparative Example 5)

The plain tablet was pulverized, and an appropriate amount of the pulverized tablet was dispersed or dissolved in a hypromellose solution (water:ethanol = 8:2).

### (Example 9)

The plain tablet was pulverized, and an appropriate amount of the pulverized tablet was dispersed or dissolved in a polyvinyl alcohol copolymer solution (water:ethanol = 8:2).

### 3.2 Test Method

An appropriate amount of each of the samples of Example 9 and Comparative Examples 4 and 5 was extracted and applied onto a white plate and left at 50°C for a whole day and night to prepare a casting film. Discoloration of each of the prepared casting films was visually observed. The evaluation of discoloration was performed according to the following criteria.

### 3.3 Evaluation Criteria

In the same manner as in 1.3, the evaluation was performed according to the four criteria: A: no discoloration; B: slight discoloration; C: some discoloration; and D: significant discoloration.

### 3.4 Test Results

The test results are shown in Table 4. From the results shown in the table, it was found that discoloration can be prevented when a polyvinyl alcohol copolymer is used.

**(Table 4)**

| | Comparative Example 4 | Comparative Example 5 | Example 9 |
|---|---|---|---|
| Tranexamic acid | ○ | ○ | ○ |
| Ascorbic acid | ○ | ○ | ○ |
| L-cysteine | ○ | ○ | ○ |
| Pyridoxine hydrochloride | ○ | ○ | ○ |
| Hypromellose | ○ | ○ | |
| PVA copolymer | | | ○ |
| Visual evaluation | C | D | A |

## Claims

1. A solid pharmaceutical preparation for oral administration comprising tranexamic acid and ascorbic acid, wherein said solid pharmaceutical preparation further contains a polyvinyl alcohol copolymer and/or a partially saponified polyvinyl alcohol.

2. The solid pharmaceutical preparation for oral administration according to claim 1, further containing L-cysteine.

3. The solid pharmaceutical preparation for oral administration according to claim 1 or 2, further containing pantothenic acid and/or pyridoxine.

4. The solid pharmaceutical preparation for oral administration according to claim 3, wherein the pyridoxine is pyridoxine hydrochloride.

5. The solid pharmaceutical preparation for oral administration according to claim 3 or 4, wherein the pantothenic acid is calcium pantothenate.

## Patentansprüche

1. Feste pharmazeutische Zubereitung zur oralen Verabreichung, die Tranexamsäuren und Askorbinsäure umfasst, wobei die feste pharmazeutische Zubereitung ferner ein Polyvinyl-Alkohol-Copolymer und/oder einen teilweise verseiften Polyvinylalkohol enthält.

2. Feste pharmazeutische Zubereitung zur oralen Verabreichung nach Anspruch 1, die ferner L-Cystein enthält.

3. Feste pharmazeutische Zubereitung zur oralen Verabreichung nach Anspruch 1 oder 2, die ferner Pantothensäure und/oder Pyridoxin enthält.

4. Feste pharmazeutische Zubereitung zur oralen Verabreichung nach Anspruch 3, wobei das Pyridoxin Pyridoxinhydrochlorid ist.

5. Feste pharmazeutische Zubereitung zur oralen Verabreichung nach Anspruch 3 oder 4, wobei die Pantothensäure Kalziumpantothenat ist.

## Revendications

1. Formulation pharmaceutique solide destinée à une administration orale, comprenant de l'acide tranexamique et de l'acide ascorbique, dans laquelle ladite formulation pharmaceutique solide contient en outre un copolymère d'alcool polyvinylique et/ou un alcool polyvinylique partiellement saponifié.

2. Formulation pharmaceutique solide destinée à une administration orale selon la revendication 1, contenant en outre de la L-cystéine.

3. Formulation pharmaceutique solide destinée à une administration orale selon la revendication 1 ou 2, contenant en outre de l'acide pantothénique et/ou de la pyridoxine.

4. Formulation pharmaceutique solide destinée à une administration orale selon la revendication 3, dans laquelle la pyridoxine est de l'hydrochlorure de pyridoxine.

5. Formulation pharmaceutique solide destinée à une administration orale selon la revendication 3 ou 4, dans laquelle l'acide pantothénique est du pantothénate de calcium.
